# EUROPEAN PATENT APPLICATION

(11) **EP 1 972 286 A2**
(43) Date of publication of application: **24.09.2008**
(21) Application number: 08250465.5
(22) Date of filing: 07.02.2008
(51) Int. Cl.: A61B 17/02, G05G 5/06

(54) **Table post holder**

(30) Priority: 08.02.2007 US 888804 P
(71) Applicant: Codman & Shurtleff, Inc., Raynham, Massachusetts 02767-0350 (US)
(72) Inventor: Agbodoe, Victor, Stoughton MA 02072 (US)
(74) Representative: Tunstall, Christopher Stephen

(57) **Abstract**

A table post holder for use during surgery includes a table connecting portion and an attachment portion. The table connecting portion has a clamp to selectively fixedly connect the table connecting portion to a table. The clamp includes a fixed clamp portion and a moveable clamp portion. An attachment portion has an opening configured to be connected to a post. An annual mating saw connection is disposed between the table connecting portion and the attachment portion. A handle is attached to the connection portion to selectively place the table connecting portion and the attachment portion in one of a fixed position with respect to each other and a moveable position. In the moveable position the attachment portion is free to rotate 360° with respect to the table connecting portion.

## Description

### FIELD OF THE INVENTION

The present invention relates to methods and devices for providing a table post.

### BACKGROUND OF THE INVENTION

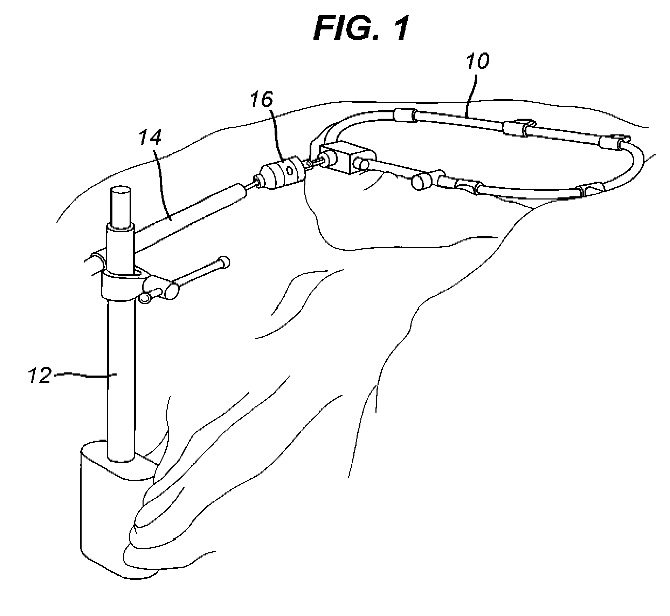

During surgery, retractors, such as those found in the BOOKWALTER™ retractor kit, which is commercially available from Codman & Shurtleff, Inc. of Raynham, MA, are often used to assist the surgeon and other operating room personnel to provide exposure to the surgical site for a broad range of surgical procedures. In surgical operations of the chest or abdomen, for example, it is often necessary to use a retraction apparatus to retain tissue away from the operative site. Typically, the retraction apparatus includes a housing member configured to lock onto a circumferential ring 10 located above the operative site (see Fig. 1). To maintain ring 10 in a fixed position, ring 10 can be connected to a support post 12 adjacent to the site by a horizontal bar 14 that has a flexible joint 16 (horizontal flex bar) to permit ring 10 to be oriented in various positions, as shown in Fig. 1. A horizontal extension arm may also be attached to the support post for supporting the circumferential ring. Within the housing member a retraction blade can usually be found for grabbing the tissue around the surgical incision. The housing member can also include a ratcheting mechanism and/or a tilting mechanism to draw the retraction blade away from the incision, thereby effecting the pulling away and/or lifting of the tissue around the incision to expose the desired surgical area. Examples of such retractor systems are disclosed in U.S. Pat. Nos. 4,254,763, 4,424,724 and 5,375,481, the disclosures of which are hereby incorporated by reference. During open surgical operations, such as, for example, open bariatric, ALIF procedures, hepatic resections, transplant procedures, abdominal aortic aneurysms, hernia repair, appendectomy and others, many different instruments are used, such as, for example, a retractor blade with ring attachment systems are used. In some of these operations, the retraction system needs to be mounted on a table, while permitting a post to be connected to the table post holder with 360 degrees of rotation. Thus, there is a need for a table post holder that permits a post to be connected to the table post holder with the flexibility to be positioned with 360 degrees of rotation.

Thus, despite these retraction systems, there continues to be a need for a table post holder that can permitting a post to be connected to the table post holder with 360 degrees of rotation.

### SUMMARY OF THE INVENTION

### BRIEF DESCRIPTION OF THE DRAWINGS

The invention will be more fully understood from the following detailed description taken in conjunction with the accompanying drawings, in which:

FIG. 1 is a plan view of a conventional retractor system;

FIG. 2 is a front view of the table post holder in accordance with the present invention;

FIG. 3 is a left side view of the table post holder;

FIG. 4 is a right side view of the table post holder and

FIG. 5 is a cross-sectional view of the table post holder.

### DETAILED DESCRIPTION OF THE INVENTION

Certain exemplary embodiments will now be described to provide an overall understanding of the principles of the structure, function, manufacture, and use of the devices and methods disclosed herein. One or more examples of these embodiments are illustrated in the accompanying drawings. Those skilled in the art will understand that the devices and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments. Such modifications and variations are intended to be included within the scope of the present invention.

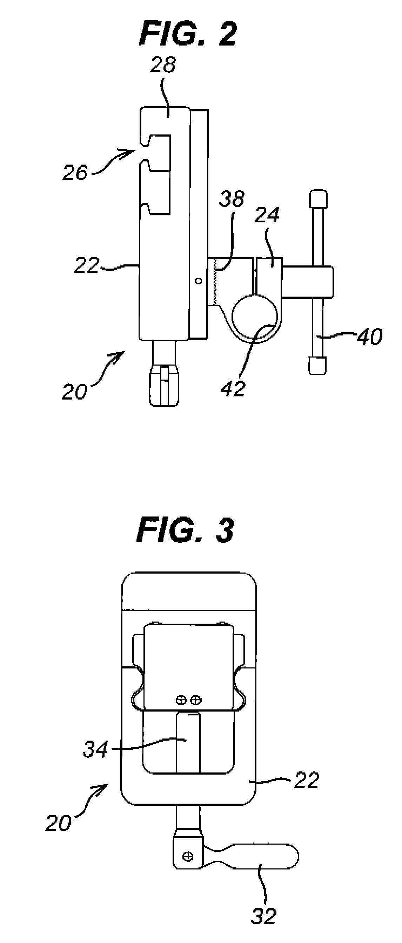

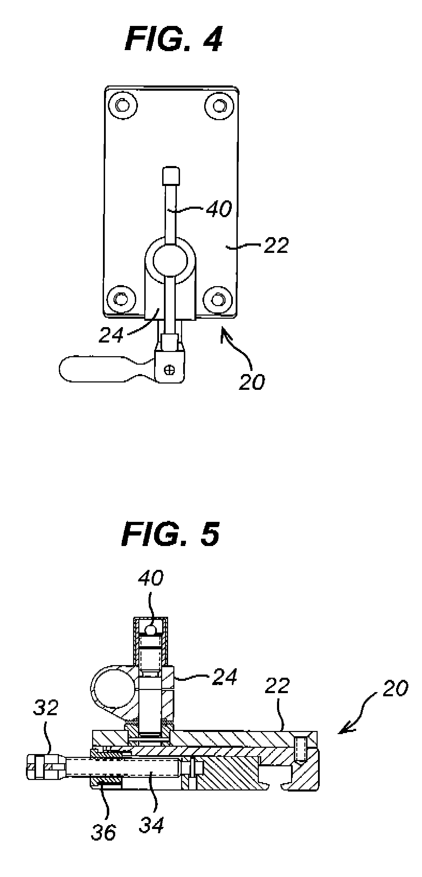

The present invention provides methods and devices for a table post holder for use with retractor systems. A table post holder 20 is illustrated in Figs. 2-5. Holder 20 includes a table connecting portion 22 and an attachment portion 24. Table connecting portion 22 includes a clamp 26 to selectively fixedly connect holder 20 to a table. Clamp 26 includes a fixed clamp portion 28 and an axially moveable clamp portion 30, which is fixedly connected to a handle 32 via a shaft 34. Shaft 34 is threaded and mates with a bushing 36 to cause axial displacement of shaft 34 upon rotation of handle 32. Thus, handle 32 can be rotated either clockwise or counter-clockwise to open clamp 26 and to securely connect table post holder 20 to a table.

Table connecting portion 22 and attachment portion 24 are selectively fixedly connected to each other by an annual mating serrated saw-like connection 38, which may also be referred to as a starburst connection. Connection 38 is selectively loosened and tightened by rotating handle 40 in either a clockwise or counter-clockwise direction. In the loosened position, attachment portion 24 is free to rotate 360 degrees with respect to table connecting portion 22. In the tightened position, however, attachment portion 24 is fixedly connected to table connecting portion 22. A post (not shown) can be connected to attachment portion 24 at opening 42 in a manner known to those skilled in the art. Thus, the table post holder 20 permits a post to be connected to the table post holder with the flexibility to be positioned with 360 degrees of rotation.

One skilled in the art will appreciate further features and advantages of the invention based on the above-described embodiments. Accordingly, the invention is not to be limited by what has been particularly shown and described, except as indicated by the appended claims. All publications and references cited herein are expressly incorporated herein by reference in their entirety.

## Claims

1. A table post holder for use during surgery comprising:
a table connecting portion having a clamp to selectively fixedly connect said table connecting portion to a table, said clamp includes a fixed clamp portion and a moveable clamp portion;
an attachment portion having an opening configured to be connected to a post;
an annual mating saw connection being disposed between said table connecting portion and said attachment portion;
a handle being attached to said connection portion to selectively place said table connecting portion and said attachment portion in one of a fixed position with respect to each other and a moveable position, in said moveable position said attachment portion is free to rotate 360° with respect to said table connecting portion.

2. The table post holder according to claim 1, wherein said handle is rotatable.

3. The table post holder according to claim 1, further comprising a second handle connected to said moveable clamp portion.

4. The table post according to claim 3, wherein said shaft is threaded.

5. The table post according to claim 4, wherein said attachment portion includes a bushing, said shaft mates with said bushing.
